# DEMANDE DE BREVET EUROPEEN

(11) **EP 1 493 349 A1**
(43) Date de publication de la demande: **05.01.2005**
(21) Numéro de dépôt: 04291631.2
(22) Date de dépôt: 29.06.2004
(51) Int. Cl.: A44B 18/00

(54) **Complexe constitué d'un film et d'un tricot ou d'un non-tisse pour auto-agrippant, notamment pour couche-culotte**

(30) Priorité: 04.07.2003 FR 0308201
(71) Demandeur: Aplix Société Anonyme, 75008 Paris (FR)
(72) Inventeur: Ducauchuis, Jean-Pierre, 44100 Nantes (FR); Marche, Thierry, 44450 La Chapelle Basse Mer (FR)
(74) Mandataire: Eidelsberg, Olivier Nathan

(57) **Abrégé**

Ensemble (1) ou complexe stratifié, comportant un film (2) et un tricot ou un non tissé fixé sur le film, le tricot, respectivement le non tissé, étant constitué d'une base formée de fils (5, 6), respectivement de filaments, et de bouches issues de la base, caractérisé en ce que au moins une partie des fils, respectivement des filaments, de la base sont ancrés dans la matière du film, et le film a un poids inférieur à 20 g/m², notamment compris entre 5 et 15 g/m².

## Description

La présente invention se rapporte à un procédé de fabrication d'un complexe ou ensemble stratifié constitué d'un film, notamment en matière plastique classique, et d'un tricot ou d'un non tissé, en particulier d'un tricot ou d'un non tissé à boucles ainsi qu'à un complexe ou ensemble obtenu par le procédé. Ce genre d'ensemble stratifié est utilisé, en particulier, pour la fabrication de parties femelles d'un autoagrippant à crochets et boucles, notamment dans le domaine des couches-culottes.

On connaît déjà, par exemple, par le brevet français n° 95 14140 au nom de la demanderesse, des ensembles stratifiés de ce genre. Le tricot y est contrecollé sur le film, soit par dépôt de colle sur le film, puis placage du tricot contre le film, soit par dépôt de colle au dos du tricot, puis laminage sur le film.

Dans le premier cas, il se pose le problème que les boucles qui sont issues du tricot, lors du placage ou calandrage du film contre le tricot, ont tendance à venir en contact avec la colle et à s'y coller. Une fois les boucles noyées dans la colle qui a ensuite durci, elles n'ont plus aucune efficacité pour accrocher dans les crochets. Pour résoudre ce problème, le brevet de la demanderesse mentionné ci-dessus prévoit de réaliser des grandes boucles, c'est-à-dire des boucles d'une dimension telle qu'elles sont en partie protégées par les fils formant la base du tricot et éventuellement le pied de la boucle voisine.

Dans le deuxième cas, comme décrit dans le brevet français n 9608259 au nom de la demanderesse, on peut certes utiliser des petites boucles et un tricot ouvert, mais la vitesse de production de l'ensemble stratifié est limitée, notamment en raison de l'application de la colle en cordons sous forme de quadrillage.

Dans un cas comme dans l'autre il est nécessaire d'utiliser des films résistants à la tension dans le sens de la machine pour supporter l'étirage lié à l'opération de calandrage.

Le document WO 99/14045 décrit un ensemble constitué d'un non tissé et d'un film. Le non tissé est fixé thermiquement par points d'ancrage sur le film, laissant un espace libre entre eux pour permettre l'accrochage de crochets d'auto-agrippant. Sa fabrication est compliquée en raison de cette fixation par points d'ancrage, d'où également une vitesse de production limitée. En outre, en raison du procédé de fabrication, il est nécessaire d'utiliser un film assez lourd. Pas plus de 30 % en longueur des fils inférieurs (du côté du film) ne sont en contact avec le film.

La présente invention vise à obtenir un complexe ou ensemble stratifié comme décrit précédemment, destiné à être utilisé comme partie femelle d'un auto-agrippant à crochets et boucles, qui puisse avoir des boucles de petite dimension par rapport au pas de l'entrelacement formant la base du tricot sans que, cependant, cela n'affecte la capacité d'accrochage des boucles dans les crochets, notamment sans que les boucles ne se noient dans la colle, et ce à une vitesse de production élevée.

Suivant l'invention, le procédé de fabrication d'un ensemble stratifié comportant un tricot ou un non tissé et un film comportant l'étape qui consiste à faire avancer un tricot ou un non tissé est caractérisé en ce qu'on extrude de la matière thermoplastique sur le tricot ou non tissé qui avance pour former le film sur le tricot ou non tissé, et à soumettre le complexe tricot ou non tissé et matière thermoplastique à un champ électrostatique pour ancrer le film en matière thermoplastique dans le tricot ou le non tissé.

Suivant l'invention, la partie femelle d'un autoagrippant, comportant un ensemble ou complexe stratifié, constitué d'un film et d'un tricot fixé sur le film, le tricot étant constitué d'une base, formée de fils, et de boucles issues de la base, est caractérisée en ce que au moins une partie des fils de la base sont ancrés dans la matière du film, et le film ayant un poids inférieur à 20 g/m², notamment compris entre 5 et 15 g/m² .

Suivant l'invention, il n'y a plus maintenant de couche de colle ou de quadrillage de cordons de colle entre le tricot et le film. Pour fabriquer cet ensemble stratifié, on extrude et on lie directement le film au tricot. Cette extrusion se fait sans pression et en utilisant l'électricité statique, de sorte que la matière plastique formant le film vient enrober, de préférence partiellement, les fils de la base du tricot, sans venir en contact avec les boucles qui se trouvent en dessous. Ainsi, une fois la matière plastique durcie, les fils sont ancrés en partie dans la matière plastique sans que les boucles n'aient été touchées. On obtient ainsi des boucles qui sont bien libres par rapport à l'entrelacement de fils formant la base. Cette extrusion peut se faire à grande vitesse et l'absence d'étape de calandrage ou de pressage fait que le film utilisé n'a pas besoin d'être épais pour supporter ces opérations de calandrage ou de pressage, la minceur du film diminuant ainsi le coût du complexe.

La présente invention se rapporte également à une partie femelle d'un autoagrippant, comportant un ensemble ou complexe stratifié, constitué d'un film et d'une couche de non tissé fixée sur le film, la couche de non tissé ayant une base, formée des filaments les plus inférieurs de la couche, et des boucles formées par les autres filaments entre eux et entre eux et les filaments les plus inférieurs, caractérisée en ce que au moins 30 %, de préférence plus de 30 %, en longueur des filaments de la base sont ancrés dans la matière du film, le film ayant un poids inférieur à 20 g/m², notamment compris entre 5 et 15 g/m².

Suivant l'invention, il n'y a plus maintenant de couche de colle ou de quadrillage de cordons de colle entre le non tissé et le film. Pour fabriquer cet ensemble stratifié, on extrude et on lie directement le film au non tissé. Cette extrusion se fait sans pression et en utilisant l'électricité statique, de sorte que la matière plastique formant le film vient enrober, de préférence partiellement, les filaments de la base, sans venir en contact avec les boucles qui se trouvent au-dessus. Ainsi, une fois la matière plastique durcie, les filaments sont ancrés en partie dans la matière plastique sans que les boucles n'aient été touchées. Cette extrusion peut se faire à grande vitesse et l'absence d'étape de calandrage ou de pressage fait que le film utilisé n'a pas besoin d'être épais pour supporter ces opérations de calandrage ou de pressage, la minceur du film diminuant ainsi le coût du complexe.

De préférence, on utilise pour la matière du film une matière ayant une bonne adhérence sur la matière constituant les fils formant la base du tricot ou du non tissé.

De préférence, de 20% à 60%, notamment de 30% à 50% de la base, est enrobée dans la matière du film.

On obtient ainsi une résistance très importante au délaminage en raison d'une combinaison d'une fixation chimique et mécanique, sans pour autant que les boucle soient atteintes.

Suivant un mode de réalisation préféré de l'invention, le film est en une matière qui peut être imprimée. Suivant l'invention, le film du complexe peut être très mince. En effet, on réalise ainsi des rouleaux d'ensembles stratifiés plus longs pour un même diamètre par rapport à l'art antérieur.

Suivant l'invention, dans un mode de réalisation préféré, dans le cas où l'on réalise des produits qui ne demandent pas à être imprimés ou respirables, le film peut avoir un poids inférieur à 10 g/m², notamment compris entre 5 g/m² et 10 g/m².

Suivant un mode de réalisation préféré de l'invention, le tricot ou le non tissé a un poids inférieur à 15 g/m², voire inférieur à 10 g/m². Des tricots ou des non tissés très fins de ce genre sont possibles car l'extrusion est réalisée sur des tricots ou non tissés faiblement tendus, le coût de fabrication du complexe étant ainsi particulièrement bas.

Suivant un mode de réalisation préféré de l'invention, la base est constituée d'un entrelacement de fils de trame et de colonnes de mailles, et les boucles sont tricotées dans l'entrelacement.

Suivant un mode de réalisation préféré de l'invention, l'ensemble stratifié comporte une couche extérieure ou plusieurs couches extérieures, notamment une couche adhésive ou autoadhésive, une couche pour assurer un maintien mécanique plus important ou une couche pour assurer une imperméabilité à certaines longueurs d'ondes.

La présente invention se rapporte également à un autoagrippant à crochets et boucles comportant une partie à crochets et une partie à boucles, la partie à boucle ayant un complexe suivant l'invention. La présente invention se rapporte aussi à une couche-culotte ayant un autoagrippant suivant l'invention.

On décrit maintenant un mode de réalisation préféré de l'invention en se reportant au dessin, dans lequel :
la Figure 1 est une vue de dessus d'un complexe suivant l'invention ;
la Figure 2 est une vue de côté d'une partie d'un complexe suivant l'invention ;

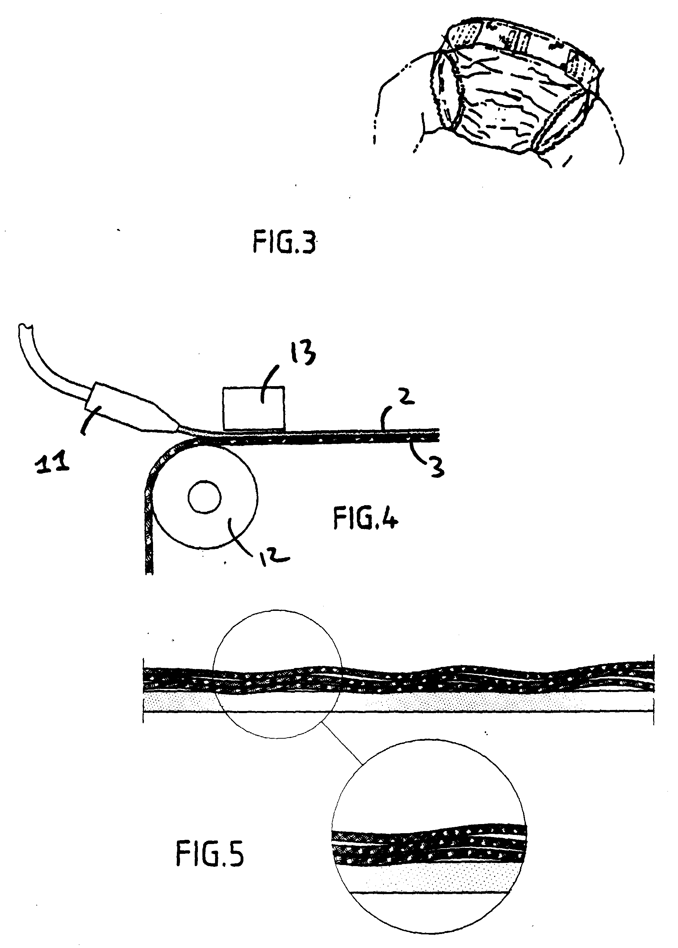

la Figure 3 est une vue d'une couche-culotte ayant un autoagrippant dont la partie boucle comporte un complexe suivant l'invention ;
la Figure 4 est une vue d'ensemble d'une installation de fabrication d'un complexe suivant l'invention ; et

La Figure 5 est une vue équivalent à celle de la Figure 2 dans le cas d'un non tissé.

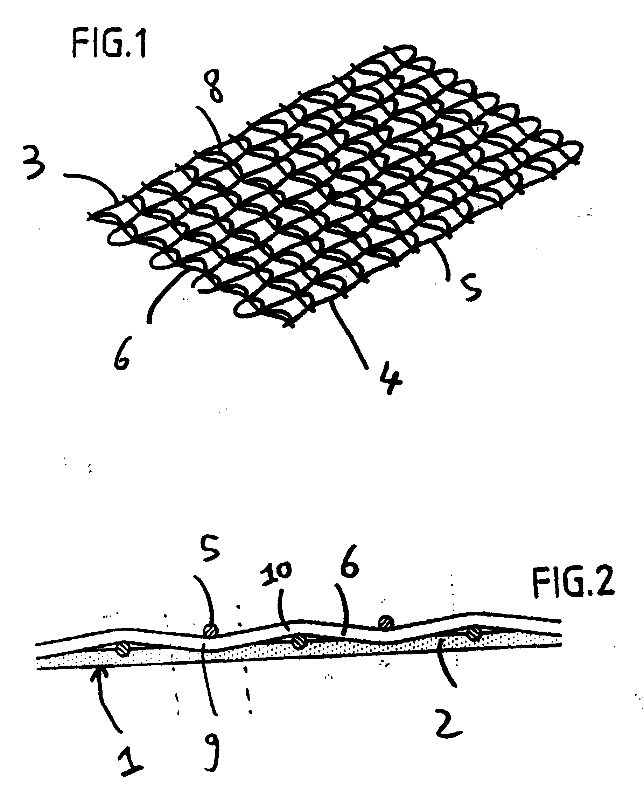

A la Figure 1, on peut voir un ensemble stratifié 1 constitué d'un film 2 et d'un tricot 3. Le tricot 3 est constitué d'une base 4 formée d'un entrelacement de fils de chaîne 5 et de colonnes de mailles 6. Cet entrelacement forme un réseau régulier, avec des mailles polygonales, à quatre côtés. Dans l'entrelacement de fils de chaîne et de colonnes de mailles, il est tricoté des boucles 8 également en réseau. Ici, la dimension des boucles est telle que lorsqu'elles sont plaquées contre la base du tricot, elles sont circonscrites par une maille de la base. En effet, il n'est pas nécessaire de prévoir des grandes boucles, c'est-à-dire des boucles ayant une dimension plus grande que celle de la maille polygonale, à quatre côtés. Cependant, si on le souhaite, on peut bien évidemment réaliser des grandes boucles.

Comme on le voit à la Figure 2 (les boucles n'y sont pas représentées pour simplifier), les fils 5, 6 de l'entrelacement de colonnes de mailles et de fils de trame sont partiellement enrobés de la matière du film 2. Un fil de trame sur deux est partiellement enrobé dans la matière plastique formant le film, tandis que les fils des colonnes de mailles comportent en alternance des tronçons 9 partiellement ancrés et des tronçons 10 libres. Comme on le voit à la Figure 2, 50 % de la circonférence des fils de trame partiellement ancrés, en section transversale, est entourée par la matière, notamment plastique, du film et 30% des tronçons partiellement ancrés sont ancrés dans la matière. Ainsi environ 40% de l'ensemble des fils formant l'entrelacement sont ancrés dans le film. Le film est en matière plastique, notamment en EVA, polypropylène, polyéthylène ou polyester. Concernant le tricot, la base peut être en polyester, polyamide ou polypropylène, tandis que les boucles peuvent être en polyamide ou polyester. Ces différentes matières ont une bonne affinité en termes d'adhérence, de sorte que les fils sont bien ancrés dans la matière et difficile à délaminer. On pourrait également prévoir que toute la circonférence du fil soit noyée dans la matière plastique. Cependant, ceci peut être plus lent à réaliser et peut entraîner, lorsque la matière déborde quelque peu vers le haut, que certaines boucles soient également prises dans la matière, ce qui n'est pas souhaitable. Par conséquent, il est préférable de régler la pression (ou débit) de la matière plastique extrudée sur le tricot de sorte que seulement partiellement les fils de tricot soient noyés dans la matière plastique du film:

Considéré dans leur longueur, les fils de l'entrelacement sont au moins en contact linéaire avec le film sur au moins 30 % de leur extension en longueur, notamment sur au moins 50 % (cas de la figure), de préférence sur au moins 75 %, par exemple sur 100 %

En outre, on peut déposer une couche supplémentaire sur le film en matière plastique. En particulier, cette couche peut être, par exemple, adhésive, notamment thermoréactivable ou autoadhésive, en vue de fixer, par exemple, l'ensemble stratifié sur une couche-culotte pour former la partie femelle d'un autoagrippant servant à fermer la couche-culotte. Bien évidemment, on peut tout simplement aussi coller l'ensemble stratifié sans prévoir cette couche supplémentaire. On peut également prévoir une autre couche simplement pour assurer une plus grande rigidité de l'ensemble et donc un meilleur maintien mécanique. On peut également prévoir une couche présentant une imperméabilité à certaines longueurs d'ondes.

Pour l'extrusion du film sur le tricot, on achemine la matière plastique par des moyens connus disposés par exemple dans l'ordre suivant : 1. Système de dosage/mélangeage des différentes matières plastiques, 2. .Extrudeuse avec vis spécifique refroidie ou non, 3. Filtre sortie extrudeuse, 4. Pompe de dosage (ou sans), 5. Filière d'enduction/extrusion. On règle l'épaisseur du film extrudé par le débit de la matière (et non comme habituellement par la géométrie de la filière).

La filière 11 est orientée préférentiellement d'un angle inférieur à 45°, notamment de 15° à 25°, par exemple 22°, avec un tricot 3 sur lequel le film 2 est déposé. Le débit de la matière doit être régulé de telle sorte que le film obtenu soit emmené par le tricot 3, qui est entraîné par un rouleau 12. La hauteur de la filière par rapport au support est réglable et dépendante des matières utilisées, des angles utilisés et de la vitesse machine. Elle est de 25 mm en ordre de grandeur.

Un système 13 de charge électrostatique type ELTEX (30 KV/200 mm) est utilisé lors de la cohésion afin d'assurer le contact intime entre le film et le support. Il n'y a donc plus de calandrage par pression mécanique et ce système sans pression permet de ne pas altérer les caractéristiques autoagrippantes intrinsèques des éléments constitutifs. Il pourra être ensuite réalisée une zone de refroidissement pour stabiliser le complexe. La vitesse de la machine est usuellement de 200 m/mn mais peut atteindre jusque 500 m/mn.

La tension de surface du support peut être traitée (corona, plasma, flammage...), si besoin pour améliorer l'adhésion.

A la place du tricot 3, on peut prévoir un non tissé constitué de filaments enchevêtrés, au moins une partie en hauteur de l'enchevêtrement étant enrobé dans la matière plastique du film.

Le non tissé, constitué de filaments enchevêtrés et empilés pour former une couche, forme une base, constituée des filaments les plus à l'intérieur ou les plus inférieurs (du côté du film), les autres filaments (dans la partie supérieure de la couche) formant des boucles permettant un accrochage de crochets d'un autoagrippant.

De préférence, la totalité de la base est en contact avec, ou est ancrée dans, le film.

De préférence, considéré suivant l'extension en longueur des filaments de la base (les filaments les plus inférieurs), au moins 30 % de la longueur de chaque filament formant la base est en contact avec, ou est ancré dans, le film, de préférence au moins 50 %, plus préférablement au moins 75 %, notamment 100 %.

## Revendications

1. Partie femelle d'un autoagrippant, comportant un ensemble (1) ou complexe stratifié, comportant un film (2) et un tricot (3) fixé sur le film, le tricot étant constitué d'une base (4) formée de fils (5, 6), et de boucles (8) issues de la base, **caractérisé en ce que** au moins une partie des fils de la base sont ancrés dans la matière du film, et le film a un poids inférieur à 20 g/m², notamment compris entre 5 et 15 g/m².

2. Partie femelle d'un autoagrippant, comportant un ensemble ou complexe stratifié, constitué d'un film et d'un non-tissé, le non-tissé étant constitué d'une base formés de filaments et de boucles issues de la base, au moins une partie des filaments de la base étant ancrés dans la matière du film et le film ayant un poids inférieur à 20 g/m2, notamment compris entre 5 et 15 g/m2, **caractérisée en ce que** de 20 à 60%, notamment de 30 à 50% des filaments de la base sont enrobés dans la matière du film.

3. Complexe suivant la revendication 1 ou 2, **caractérisé en ce que** l'on utilise pour la matière du film (2) une matière ayant une bonne adhérence sur la matière constituant les fils (5, 6) formant la base du tricot ou les filaments formant la base du non tissé.

4. Complexe suivant la revendication 1 ou 3, **caractérisé en ce que** de 20 à 60%, notamment de 30 à 50% des fils de la base sont enrobés dans la matière du film.

5. Complexe suivant l'une des revendications précédentes, **caractérisé en ce que** le film (2) a un poids inférieur à 20 g/m2, notamment compris entre 10 et 15 g/m², et est en une matière qui peut être imprimée.

6. Complexe suivant l'une des revendications 1 à 4, **caractérisé en ce que** dans le cas où l'on réalise des produits qui ne demandent pas à être imprimés ou respirables, le film a un poids inférieur à 10 g/m², notamment compris entre 5 g/m² et 10 g/m².

7. Complexe suivant l'une des revendications précédentes, **caractérisé en ce que** le tricot, respectivement le non-tissé, a un poids inférieur à 15 g/m², voire inférieur à 10 g/m².

8. Complexe suivant la revendication 1, 3, 4, 5, 6 ou 7, **caractérisé en ce que** la base est un tricot constitué d'un entrelacement de fils de trame et de colonnes de mailles, et les boucles sont tricotées dans l'entrelacement.

9. Complexe suivant l'une des revendications précédentes, **caractérisé en ce que** l'ensemble stratifié comporte une couche extérieure ou plusieurs couches extérieures, notamment une couche adhésive ou autoadhésive, une couche pour assurer un maintien mécanique plus important ou une couche pour assurer une imperméabilité à certaines longueurs d'ondes.

10. Autoagrippant à crochets et boucles, comportant une partie à crochets et une partie à boucles, la partie à boucles comportant un complexe suivant l'une des revendications 1 à 8.

11. Couche-culotte comportant un autoagrippant, pour la fermeture de la couche au niveau de la taille, suivant la revendication 9.
